# EUROPEAN PATENT SPECIFICATION

(11) **EP 4 090 971 B1**
(45) Date of publication and mention of the grant of the patent: **26.02.2025**
(21) Application number: 21703812.4
(22) Date of filing: 17.01.2021
(51) Int. Cl.: G01N 33/50, G01N 33/68

(54) **METHOD FOR SEPARATION OF SPERM WITH UNDAMAGED INTACT HEADS FROM SPERM WITH DAMAGED HEADS AND SOMATIC CELLS**
VERFAHREN ZUR TRENNUNG VON SPERMIEN MIT UNBESCHÄDIGTEM INTAKTEM KOPF VON SPERMIEN MIT BESCHÄDIGTEM KOPF UND SOMATISCHEN ZELLEN
PROCÉDÉ DE SÉPARATION DE SPERME AVEC TÊTES INTACTES NON ENDOMMAGÉES ET DE SPERME AVEC TÊTES ENDOMMAGÉES ET CELLULES SOMATIQUES

(30) Priority: 17.01.2020 EP 20152470
(43) Date of publication of application: 23.11.2022
(73) Proprietor: Biotechnologicky ustav AV CR, v.v.i., 25250 Vestec (CZ); Molecule 46 s.r.o., 19800 Praha 9 (CZ); Ceská zemedelská univerzita v Praze, 165 00 Praha 6 (CZ)
(72) Inventor: KOMRSKOVA, Katerina, 14900 Praha 4 (CZ); POSTLEROVA, Pavla, 25264 Velke Prilepy (CZ); FROLIKOVA, Michaela, 25101 Ricany (CZ); FOROSTYAK, Serhiy, 18000 Praha 8 (CZ); SIMONIK, Ondrej, 75366 Hustopece nad Becvou (CZ)
(74) Representative: Harber IP s.r.o.
(86) International application number: PCT/CZ2021/050004
(87) International publication number: WO 2021/143958

(56) References cited:
- J. F. ODHIAMBO ET AL: "Increased Conception Rates in Beef Cattle Inseminated with Nanopurified Bull Semen", BIOLOGY OF REPRODUCTION, vol. 91, no. 4, 17 September 2014 (2014-09-17), US, pages 97 - 97, XP055239594, ISSN: 0006-3363, DOI: 10.1095/biolreprod.114.121897
- MARTÍNEZ-SOTO JUAN CARLOS ET AL: "Assessment of two thawing processes of cryopreserved human sperm in pellets", CRYOBIOLOGY, ACADEMIC PRESS INC, US, vol. 63, no. 3, 23 August 2011 (2011-08-23), pages 131 - 136, XP028597748, ISSN: 0011-2240, DOI: 10.1016/J.CRYOBIOL.2011.08.001
- SILVA P F N ET AL: "Detection of damage in mammalian sperm cells", THERIOGENOLOGY, LOS ALTOS, CA, US, vol. 65, no. 5, 15 March 2006 (2006-03-15), pages 958 - 978, XP027930357, ISSN: 0093-691X, [retrieved on 20060315]
- PETER M JOHNSON ET AL: "Rapid sperm acrosome reaction in the absence of acrosomal CD46 expression in promiscuous field mice (Apodemus)", JOURNALS OF REPRODUCTION & FERTILITY, vol. 134, no. 6, 1 December 2007 (2007-12-01), GB, pages 739 - 747, XP055708907, ISSN: 1470-1626, DOI: 10.1530/REP-07-0363

## Description

### Field of Art

The present invention relates to a method of separation of sperm with undamaged intact heads from sperm with damaged heads, as well as from somatic cells and cell debris, in cell-containing samples obtained from an animal, such as ejaculate or semen sample. Without these further steps being part of the invention, this method enables, for example, to use the separated sperm with intact heads in assisted reproduction, animal breeding programs and veterinary medicine to enhance the fertility success.

### Background Art

Artificial fertilization is a method widely used in the treatment of reproductive disorders by means of assisted reproduction as well as in agriculture, animal breeding programs and veterinary medicine. In all applications of artificial fertilization, the sperm quality is of crucial importance for achieving high fertilization success rates. Technologies for improvement of sperm quality are therefore being developed and they are of high priority. Some of the available methods for sperm quality improvement rely on health regimens and/or dietary improvements implemented by the males. Other methods focus on improving of sperm quality by enriching the sample in healthy sperm. A representative of such method is the method described in US 2017/0191029 in which a sperm sample is contacted with an insoluble carrier, which binds to an indicator of impaired sperm membrane integrity or impaired sperm motility. However only commercially available beads were tested in this document, some of which allowed achieving approximately 20% increase in motility, where for example, anti-rabbit IgG seemed to improve the ratio of healthy sperm.

CD46 (membrane cofactor protein) is membrane-associated glycoprotein that is present in human cells including sperm and in cells of other mammals. In somatic cells, CD46 is present on their surface and it plays a key role in protecting host cells from complement attack - a mechanism which is also used by tumour cells. Another role of CD46 protein is activation of several intracellular pathways in different types of cells including T-lymphocytes. Several pathogens use it as a receptor enabling them to enter the cell. In sperm, CD46 is localized on the head, specifically in an acrosome, and it only becomes surface exposed after a sperm acrosome reaction. The acrosome may be damaged, deformed or disrupted in non-physiological way. If the acrosome is damaged, the sperm is unable to penetrate the egg surroundings and fertilize the egg. The presence of damaged sperm, as well as somatic cells of any kind in the semen is not favourable for successful fertilization.

The role of CD46 in fertilization was already noticed by using monoclonal antibodies to the first short consensus repeat (SCR1) ectodomain of CD46, which resulted in blocking the complement-independent interaction of human sperm with zona-free oocytes/eggs *in vitro.* The recent discovery of a CD46 new human physiological ligand Jagged-1, which is highly expressed by the oocytes/eggs, strengthens the CD46 role in the initial steps of the sperm interaction with oolema (egg plasma membrane). CD46^{-/-} deficient mouse males show a higher rate of spontaneous acrosome reaction compared to wild type males. This finding led to the theory that CD46 plays a role in the stabilization of the acrosomal membrane and consequently the whole acrosome region. CD46 was reported to have a reduced expression in humans with idiopathic infertility.

In humans, the CD46 protein is expressed in all cells except erythrocytes and exists predominantly in four different isoforms - these isoforms are the products of alternative splicing of a single gene. They consist of four extracellular domains on the amino terminus called short consensus repeats (SCRs), a serine-threonine-proline (STP)-rich area, transmembrane domain (TM), intracytoplasmic anchor and one of two forms of cytoplasmic tail (CYT-1 of 16 amino acids and CYT-2 of 23 amino acids) that arise by alternative splicing at carboxyl terminus. The SCR1, 2 and 4 domains are sites of N-linked glycosylation. The STP region is a site of extensive O-linked glycosylation and is composed of 14 or 29 amino acids. The size of the STP region depends on the presence of STP exon B that can be possibly spliced out. Absence of STP exon B leads to lower molecular weight protein isoforms of CD46, while its presence leads to CD46 higher molecular weight isoforms. However, an even lower molecular weight hypoglycosylated isoform of CD46 is expressed in mammalian sperm. In humans, polymorphisms of CD46 gene were detected in normal individuals with both restriction enzymes, HindIII and EcoRI.

Martinez-Soto J.C. et al.: Cryobiology, 2011, 63(3), pp. 131-136; Silva P.F.N. et al.: Theriogenology, 2006, 65(5), pp. 958-978; Johnson P.M. et al.: Journals of Reproduction and Fertility, 2007, 134(6), pp. 739-747 disclose CD46 as a marker of completed acrosome reaction.

The document Odhiambo J.F. et al.: Biology of Reproduction, 2014, 91(4):97, pp. 1-10, discloses PNA-lectin conjugate or anti-ubiquitin antibody as selection markers for viability of sperm.

### Disclosure of the invention:

The present invention is a novel method for separation of sperm with undamaged intact heads, exploiting the fact that sperm with undamaged intact heads do not present CD46 on their surface in contrast to sperm with damaged heads (partially or totally damaged acrosomes) and the majority of somatic cells, as well as cell debris, which present CD46 on their surface; and on a novel concept of using CD46 as a selector in a simple, fast and effective method.

The present invention provides a method of separation of sperm cells with undamaged intact heads from sperm cells with damaged heads and/or somatic cells and/or cell debris in a sperm sample obtained from an animal, comprising the steps of:
- providing an anti-CD46 antibody bound to a carrier and/or to a tag;
- contacting the sperm sample with the anti-CD46 antibody bound to the carrier and/or to the tag in order to bind CD46 presenting cells and/or cell debris from the sperm sample to the anti-CD46 antibody;
- removing the CD46 presenting cells bound via the anti-CD46 antibody to the carrier and/or to the tag to obtain a sperm sample free of CD46 presenting cells, wherein CD46 presenting cells include sperm cells with damaged heads, somatic cells, and cell debris.

The present invention is based on utilization of the fact that the CD46 molecule (official full name provided by HGNC - HUGO Gene Nomenclature Committee; http://www.genenames.org/cgi-bin/gene_symbol_report?match=CD46 https://www.ncbi.nlm.nih.gov/gene/4179) is not presented on the surface of undamaged (undisrupted, intact) sperm heads. On the other hand, the sperm with damaged heads (in particular acrosomes) and somatic cells and cell debris present CD46 molecules on their surface. Thus, using anti-CD46 antibody as a selector, unbound sperm with undamaged intact healthy heads can be separated from the sperm marked or selected for damaged heads (namely acrosomes) as well as from somatic cells and debris that are present in a sperm sample, such as an ejaculate or any other sperm containing sample. This allows to separate the undisrupted sperm with undamaged intact heads for further use, and to remove the disrupted sperm with damaged heads, somatic cells and debris from a sperm sample.

The animal is preferably a vertebrate such as a mammal or a bird.

The term "damaged sperm", "sperm with damaged heads", "sperm with disrupted heads" in this description is intended to designate sperm cells with partially or totally damaged heads, namely acrosomes, so that CD46 is presented on the surface of the sperm cells and/or is accessible for the anti-CD46 antibody.

The term "sperm with undamaged intact heads" or "undisrupted sperm cells" or "undamaged sperm cells" or "sperm cells with undisrupted heads" or "sperm cells with undamaged heads" designates sperm cells with intact and undisrupted heads, so that CD46 is not presented on the surface of the sperm cells and/or is not accessible for the anti-CD46 antibody.

The term "cell debris" is characterized as cellular components and fragmented remnants of dead or damaged cells or tissues.

The term "sperm sample" includes a sample containing a mixture of cells including sperm cells. Preferably, the sperm sample is a sperm-containing sample such as an ejaculate, or a pre-treated sperm sample, or a semen sample or a pre-treated semen sample. The sperm sample used in the present invention is typically a sperm sample which has not yet been contacted with an egg, i.e., the sperm cells have not yet undergone the acrosome reaction.

The terms "binding" and "bound" include in particular conjugation, covalent binding or non-covalent binding.

The invention enables to separate sperm cells with undamaged intact heads from sperm cells labeled by anti-CD46 antibody for damaged heads (namely damaged acrosome), as well as from somatic cells and cell debris that are present in the sample. The sample may be a sperm sample including ejaculate or semen sample, or a pre-treated sperm sample such as pre-treated ejaculate or a pre-treated semen sample. The pre-treatment may include removal of seminal fluid and/or tissues and/or debris from the ejaculate or from the semen sample or from fresh or conserved sperm, e.g. by density gradient centrifugation or by a swim-up, or by any other suitable method.

Without these further steps being part of the invention, the sample containing only sperm cells with undamaged intact heads may further be used in assisted reproduction techniques, including *in vitro* fertilization, intracytoplasmic sperm injection etc., in animal breeding programs and/or in veterinary medicine.

The method of present invention may in some embodiments contain the following steps:
- designing at least one immunization peptide for anti-CD46 antibody production;
- preparing an anti-CD46 antibody;
- binding the anti-CD46 antibody to a carrier and/or to a tag;
- contacting the anti-CD46 antibody bound to the carrier and/or to the tag with a sperm sample in order to bind CD46 presenting cells, including sperm cells with damaged heads and/or somatic cells and/or cell debris;
- separating the sperm cells with damaged heads and/or somatic cells and/or cell debris bound to the carrier and/or to the tag via the anti-CD46 antibody from the sperm sample;
thereby obtaining two cell fractions: 1) sperm cells with undamaged intact heads and 2) sperm cells with damaged heads and/or somatic cells and/or cell debris.

The method thus results in a selection and separation of sperm cells with damaged heads and/or somatic cells and/or cell debris from the fraction of the sample not presenting CD46.

The "fraction of the sample not presenting CD46" or the "sperm sample free of CD46 presenting cells" are used herein as synonyms. This fraction of the sample may include sperm cells with undamaged intact head and/or acellular components of the sample and/or red blood cells.

Without these further steps being part of the invention, any obtained fraction, i.e., the fraction of the sample not presenting CD46, or the fraction of CD46-presenting cells bound to the carrier and/or to the tag, can be used for further processing as needed, for example in artificial fertilization technologies, animal breeding programs, medical applications, for storage purposes, or research.

The CD46 peptides for anti-CD46 antibody production can be the whole CD46 (molecule/protein/peptide/ligand/receptor) or its modifications, isoforms and transcript variants (synthetically prepared substances which contain/incorporate a part of the sequence of the molecule) and derivatives (synthetically prepared substances which constitute non-functional subunit of the molecule).

The anti-CD46 antibody may be a commercially available antibody or an antibody produced by known methods. The antibodies may be monoclonal or polyclonal, conjugated or unconjugated, directly or indirectly prepared antibodies against CD46. The anti-CD46 antibodies may be prepared by any suitable method, including, for example, proteomic synthesis, hybridoma cell line technology and immunization by a tissue, cells, whole protein, extracellular part, cytoplasmic tail or peptides with/without post-translational modifications. The anti-CD46 antibodies may also be or include synthetic antibodies or derivatives of synthetic antibodies.

For example, the anti-CD46 antibody may be prepared by a method including immunization of animals and/or cells by immunization peptides, which are derived from individual species. In some embodiments, the immunization peptide may be the peptide PFEAMELKGTPKLY (SEQ ID NO. 1).

The carrier may be a substance or device capable of binding the anti-CD46 antibody or part thereof. The carrier may preferably be in a solid form, in a form of solution or in a form of gel. Examples of suitable carriers include: beads, magnetic beads, polymeric substrates (e.g. polyacrylamide, polystyrene, polycarbonate), cellulosic substrates (e.g., agarose, sepharose), metallic substrates, magnetic or magnetizable carriers. Carriers are typically materials capable of covalent or non-covalent, specifically or non-specifically, directly or indirectly in single or multiple steps binding of the anti-CD46 antibody. The carriers may be applied by coating or immobilization on the surface of materials or devices. In some embodiments, the carriers or the antibodies or the binding ligands may be coated or immobilized on the inner surface of a vessel, such as a well, well plate, beaker, capillary or test tube (if antibodies or ligands are coated or immobilized on the inner surface of a vessel, then the vessel acts as a carrier).

The contacting step of anti-CD46 antibody with a carrier is preferably carried out at about room temperature, in a physiological buffer (such as phosphate buffer, physiological saline solution). The anti-CD46 antibody(ies) bound to the carrier forms an anti-CD46 antibody-carrier complex.

The anti-CD46 antibody-carrier complex allows selection and separation of sperm cells with damaged heads and/or somatic cells and/or cell debris from sperm cells with undamaged intact heads from an ejaculate or from a sperm sample in general. Selection is based on presence or absence of CD46 labeling (meaning absence of CD46 labeling on the surface of sperm with undamaged intact heads).

The tag bound to the anti-CD46 antibody may be selected from fluorophores, chromophores, quantum dots, oligonucleotides, magnetic particles, coated magnetic particles (e.g., polymer-coated magnetic particles), His-tag, MBP-tag, GST-tag, CBP-tag, and Strep-tag. Methods of tagging of antibodies are known in the art.

Magnetic particles and coated magnetic particles may preferably have the size from 1 to 3000 nm, more preferably from 1 to 500 nm, or from 1 to 200 nm.

In case of use of the anti-CD46 antibody-carrier complex, the removal of the CD46 presenting cells is typically based on the removal of the carrier to which the CD46 presenting cells are bound via the anti-CD46 antibody. In case of use of the tagged anti-CD46 antibody (i.e., an anti-CD46 antibody bound to a tag), the removal of the CD46 presenting cells is typically based on cell sorting based on the presence or absence of the tag.

The carrier separation techniques or separation techniques based on the presence or absence of the tag include methods capable to separate carrier comprising anti-CD46 antibody with or without the CD46 presenting cells and methods capable to separate tagged cells from untagged cells. The method may be direct/indirect, passive/active, gradient, biological, biochemical, physical, chemical, enzymatic, non-enzymatic, mechanical. The method may preferably be selected from centrifugation, sedimentation, flow separation methods such as FACS, capillary flow separation, separation on chips, or methods using a magnetic force in case of magnetic or magnetizable carriers and/or tags, for example MACS or magnetic separation. The removal step may also be performed by collecting the fraction of the sample containing the unbound sperm with undamaged intact heads, for example when the carrier is a vessel.

The method of the present invention is performed *in vitro* or *ex vivo.*

### Brief description of Figures

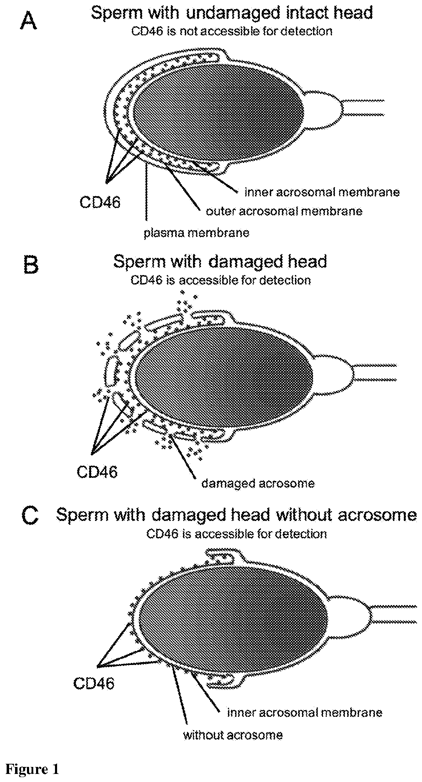

Figure 1: Schematic model of a sperm: (A) sperm with an undamaged intact head and localization of CD46 inside the intact head, which is not accessible for the antibody nor CD46-binding ligand. (B, C) sperm with the damaged head, wherein CD46 can be detected by a species specific antibody or CD46-binding ligand. (B) a partial membrane damage and partial acrosome damage. (C) an excessive head damage with a loss of acrosome.

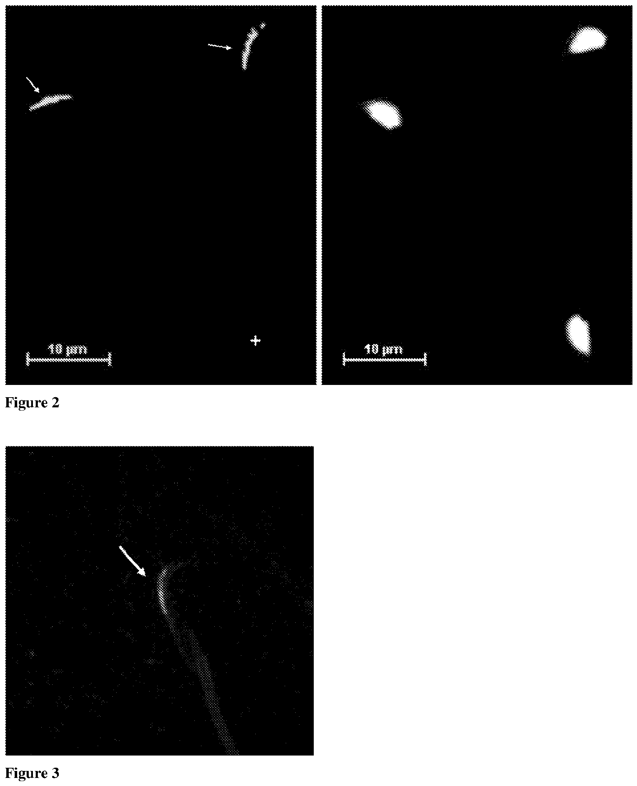

Figure 2: Mouse epididymal sperm labeled by the anti-CD46 antibody and by Hoechst for the nucleus. Left panel: white signal (arrow) shows damaged sperm labeled by the anti-CD46 antibody; no signal (cross) shows an undamaged sperm with no labeling. Right panel: white signal shows all the sperm in the picture when the labeling for nucleus was used. (The mouse photo documentation is representative for the technology used on all the animals.)
Figure 3: An image of an acrosome of a rat sperm with damaged head, labeled by anti-CD46 antibody.

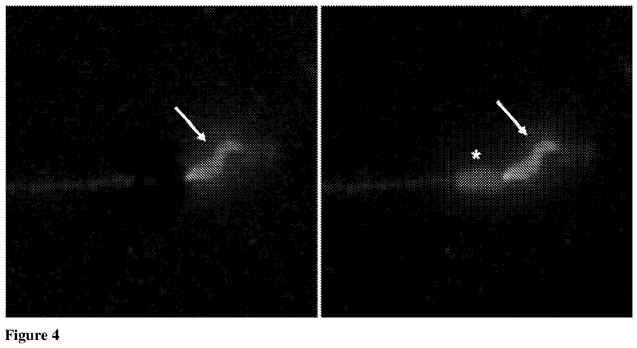

Figure 4: An image of an acrosome of a zebra finch sperm with damaged head, labeled by anti-CD46 antibody. The asterisk marks the nucleus of the sperm.

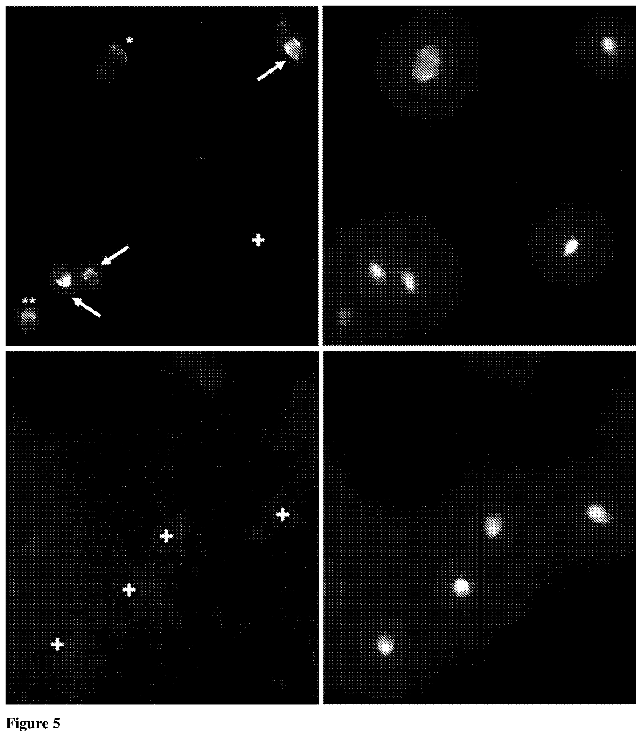

Figure 5: CD46 detection of damaged sperm, somatic cells and cell debris using fluorescent microscope. Top left panel: anti-CD46 antibody labels sperm cells with damaged heads (arrows) and somatic cells (*) and cell debris (**). Sperm cells with intact undamaged heads (+) are not labeled by anti-CD46 antibody. Top right panel: all present sperm, somatic cells and debris labeled by Hoechst. Bottom left panel: Sperm cells with intact undamaged heads are not labeled by anti-CD46 antibody (+). Bottom right panel: Sperm with intact undamaged heads labeled by Hoechst. (The documentation is representative for the technology used on all the animals.)

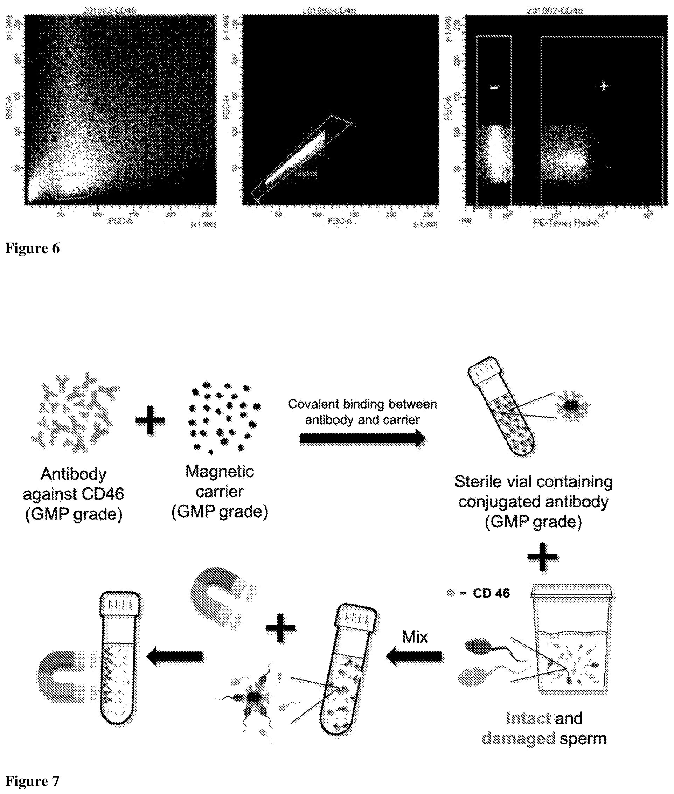

Figure 6: CD46 detection of damaged sperm, somatic cells and cell debris using Flow Cytometer. Left panel: population of cells after gating (marked by polygon). Middle panel: visualization of selected singlets. Right panel: Sperm with intact undamaged heads are not labeled by anti-CD46 antibody (-). Sperm with damaged heads are labeled with anti-CD46 antibody (+).
Figure 7: Schematic model of sperm magnetic selection example: Ejaculate sample incubation with an antibody against CD46 conjugated to a magnetic carrier. Damaged sperm and non-spermatic cells bound to the carrier via the antibody are attracted to the magnet. Intact sperm are simply transferred by a pipette and ready for further utilization.

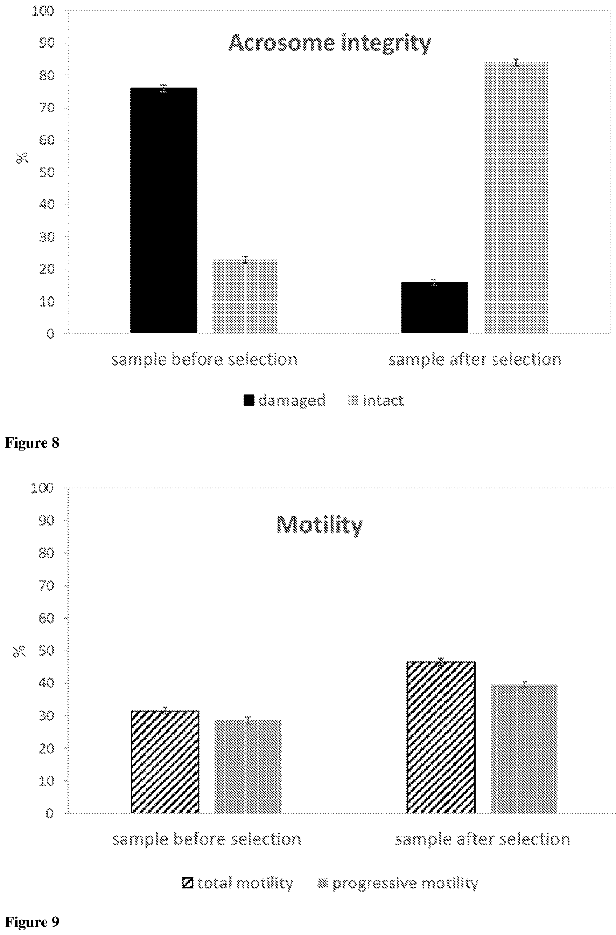

Figure 8: Sperm sample quantitative evaluation before and after selection using anti-CD46 antibody examined for acrosome integrity and damage. Sperm sample before selection contains 75% sperm with damaged acrosomes and 25% sperm with intact acrosome. Intact sperm samples after magnetic selection using CD46 marker contains 18% sperm with acrosome damage and 82% of sperm with intact undamaged heads. N(samples) = 10, error bars represent standard error of the mean (SEM).
Figure 9: Sperm sample quantitative evaluation before and after selection using anti-CD46 antibody examined for total and progressive motility by Computer Assisted Sperm Analysis (CASA). Sperm sample before selection contains 32% sperm displaying total motility, 30% of sperm with progressive motility, which in total represent 62% of motile sperm. Intact sperm samples after selection using CD46 marker contains 48% sperm detected with total motility and 44% of sperm with progressive motility, which in total represent 92% of motile sperm. N(samples) = 10, error bars represents standard error of the mean (SEM).

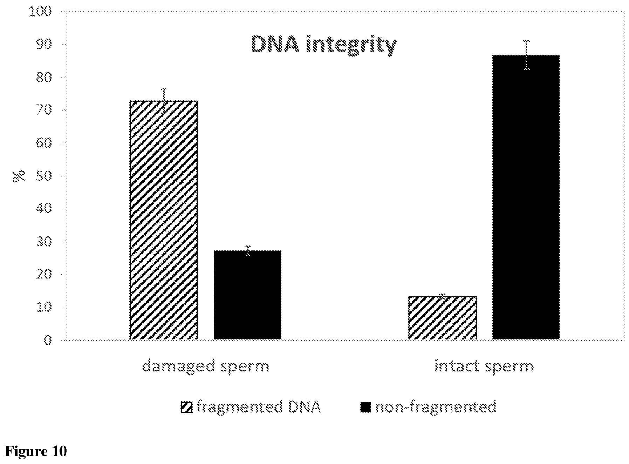

Figure 10: Sperm sample quantitative evaluation after selection using anti-CD46 antibody examined for DNA integrity by TUNEL assay in fraction with damaged sperm and fraction with intact sperm. Damaged sperm sample contains 73% sperm positive for DNA fragmentation and 27% of sperm negative for DNA fragmentation. Intact sperm samples after selection using CD46 marker contains 13% sperm positive for DNA fragmentation and 87% sperm negative DNA fragmentation showing no detectable DNA damaged. N(samples) = 10, error bars represents standard error of the mean (SEM).

### Examples of carrying out the invention

### Example 1: Differentiation of sperm with undamaged intact heads from sperm with damaged heads

Mouse epididymal sperm were labeled for nucleus by Hoechst, and for the head integrity by an anti-CD46 primary antibody (clone MM10, HM1118, Hycult Biotech, Uden, Netherland) visualized by a secondary fluorescent antibody (anti-donkey Alexa Fluor 488, Molecular Probes, Prague, Czech Republic). The Hoechst labeling showed all the cells present in the sample, while the labeling by anti-CD46 antibody showed only sperm cells with damaged heads.

Figure 2 shows an image obtained from the sample - in the left panel, labeling by the anti-CD46 antibody shows the damaged heads of sperm cells. In the right panel, labeling by Hoechst shows all the cells present in the picture. The position of the sperm cell with the undamaged intact head is marked by a cross in the left panel, for better understanding of the images by the reader.

### Example 2: Differentiation of sperm with undamaged intact heads from sperm with damaged heads, somatic cells and cell debris

Sperm cells in fresh human ejaculate were labeled for nucleus by Hoechst, and for the head integrity by an anti-CD46 primary antibody (clone M177, sc-52647, Santa Cruz Biotechnology, Inc. ) visualized by a secondary fluorescent antibody (anti-mouse Alexa Fluor 488, Molecular Probes, Prague, Czech Republic). The Hoechst labeling showed all the cells present in the sample, while the labeling by anti-CD46 antibody showed only sperm cells with damaged head.

Figure 5 shows an image obtained from the sample - Top left panel: anti-CD46 antibody labels sperm cells with damaged heads (arrows) and somatic cells (*) and cell debris (**). Top right panel: all present sperm, somatic cells and debris labeled by Hoechst. Bottom left panel: Sperm cells with intact undamaged heads are not labeled by anti-CD46 antibody (+). Bottom right panel: Sperm with intact undamaged heads labeled by Hoechst. The position of the sperm cell with undamaged intact head has been marked by a cross in the top and bottom left panels, for better understanding of the images by the reader.

### Example 3: Preparation of anti-CD46 antibody

A polyclonal anti-CD46 antibody suitable for a number of animal species was prepared as follows:
The polyclonal antibody was prepared on demand by a commercial provider, using immunization of a male New Zealand White (NZW) rabbit. The immunization peptide was PFEAMELKGTPKLY (SEQ ID NO. 1). To prepare the primary injection, 0.75 ml Freunds incomplete adjuvant (FIA) (Sigma-Aldrich, Dorset, UK) was added to 0.75 ml 800 µg/ml peptide-KLH (dissolved in sterile PBS; KLH = keyhole limpet hemocyanin). The mixture was sonicated on ice until a thick stable emulsion developed that did not disperse on the surface of water. The NZW rabbit was injected with a total of 1 ml of the peptide-KLH adjuvant mixture (400 µg) at four subcutaneous sites. Five booster injections were subsequently given over a period of four months. Booster injections comprised of 1ml of 140 µg/ml peptide-KLH adjuvant mixture injected at four subcutaneous sites.

20 ml blood was extracted from the marginal ear vein of the NZW rabbit when collecting pre-immune serum (day 0) and test bleeds (days 39, 46, 53, 68, 81, and 103). 80 ml was extracted during the terminal bleed (day 112). The blood clot was loosened from the side of the universal tube and incubated at 37°C for 1 hour. The universal tube was centrifuged at 7500g for 10 minutes and the serum collected. This step was repeated twice. The serum was incubated at 56°C for 30 minutes to inactivate heat-labile complement components. Serum was stored at -80°C. Rabbit pre-immune serum (day 0) and rabbit anti-CD46 peptide polyclonal antiserum (day 112) were affinity-purified against the non-conjugated peptide immobilised using a SulfoLink kit (Perbio Science, Cramlington, UK) according to the protocol provided. Approximately 0.5 mg of free peptide was immobilized to an agarose gel support via a reaction between iodoacetyl groups on the coupling gel and the N-terminal cysteine residue on the peptide. The column was washed and non-specific binding sites on the gel blocked by incubation with L-cysteine. Excess L-cysteine was eluted by washing. The column was incubated with serum and washed to remove non-bound serum proteins. Bound proteins were eluted using 100 mM glycine buffer (pH 2.5-3.0) (Sigma-Aldrich, Dorset, UK) and 1ml fractions were collected. The concentration of the affinity-purified serum was ascertained by absorbance at 280 nm (molar extinction coefficient of 1.4).

### Example 4: General procedure for separation of sperm with undamaged intact heads from sperm with damaged heads

Material: Cell sample suspension (ejaculate or semen sample), primary species specific antibody anti-CD46 conjugated to a carrier or with a tag.

Method: After a specie-dependent liquefaction of the ejaculate or semen sample, without using any separation method, the ejaculate or semen sample is incubated for 30 minutes with the anti-CD46 antibody conjugated to the carrier or conjugated with the tag, at room temperature, in a physiological buffer (PBS, phosphate buffer saline). Standard procedures and materials were used for binding the antibody to the carrier or to the tag. The incubation of the anti-CD46 antibody-carrier complex or anti-CD46 antibody-tag with the ejaculate or semen sample was followed by separation of the carrier or the tag with the conjugated anti-CD46 antibody to which the CD46 presenting cells are bound. The remaining unbound sperm fraction with undamaged intact heads were transferred to a new container for further use in assisted reproduction (artificial insemination) such as intrauterine insemination (IUI), *in vitro* fertilization (IVF) or intracytoplasmic sperm injection (ICSI), storage purpose, medicine, or any other technique or research.

### Example 5: General procedure for separation of sperm with undamaged intact heads from sperm with damaged heads (cryopreserved sample)

Material: Cryopreserved sample (animal ejaculate or animal semen sample), primary species specific antibody anti-CD46 conjugated to a carrier.

Method: After a sample thawing, the sample is incubated for 30 minutes with the anti-CD46 antibody conjugated to the carrier, at room temperature, in a physiological buffer (PBS, phosphate buffer saline); followed by separation of the carrier with the conjugated anti-CD46 antibody to which the CD46 presenting cells are bound. The remaining unbound sperm fraction with undamaged intact heads were transferred to a new container for further use in assisted reproduction (artificial insemination) such as intrauterine insemination (IUI), *in vitro* fertilization (IVF) or intracytoplasmic sperm injection (ICSI), storage purpose, medicine, or any other technique or research.

### Example 6: Separation of sperm with undamaged intact heads from sperm with damaged heads from the mouse (Mus musculus sp.) semen sample

The anti-mouse CD46 antibody (clone MM10, HM1118, Hycult Biotech, Uden, Netherland) is bound to magnetic beads as a carrier, at room temperature, in a physiological buffer (phosphate buffer) and forms an anti-CD46 antibody-carrier complex. The complex is added to a mouse semen sample, the procedure of Example 4 was followed. All the CD46 presenting sperm with damaged heads and all somatic cells, mainly white blood cells, and cell debris are bound to the anti-CD46 antibody-carrier complex and further separated by magnetic field, using a magnetic stand. The unbound sperm with undamaged intact heads remain in the suspension.

The same procedure was repeated with the polyclonal anti-CD46 antibody produced according to Example 3. Also with this antibody, all CD46 presenting cells (sperm with damaged heads and all somatic cells, mainly white blood cells, and cell debris) were bound to the carrier via the antibody and removed from the sample by magnetic field. The unbound sperm with undamaged intact heads remained in the suspension.

### Example 7: Separation of sperm with undamaged intact heads from sperm with damaged heads from the rat (Ratus sp.) semen sample

The anti-rat CD46 antibody (clone MM10, HM1118, Hycult Biotech, Uden, Netherland) is bound to agarose beads as a carrier, at room temperature, in a physiological buffer (PBS, phosphate buffer saline). The procedure of Example 4 was followed. All the CD46 presenting sperm with damaged heads and all somatic cells, mainly white blood cells, and cell debris are bound to the anti-CD46 antibody-carrier complex and further separated by centrifugation. The unbound sperm with undamaged intact heads remain in the suspension.

The same procedure was repeated with the polyclonal anti-CD46 antibody produced according to Example 3. Also with this antibody, all CD46 presenting cells (sperm with damaged heads and all somatic cells, mainly white blood cells, and cell debris) were bound to the carrier via the antibody and removed from the sample by centrifugation. The unbound sperm with undamaged intact heads remained in the suspension.

### Example 8: Separation of sperm with undamaged intact heads from sperm with damaged heads from the pig (Sus sp.) ejaculate

The anti-pig CD46 antibody (clone JM6C11, MCA2262GA, Bio-Rad Laboratories, Hercules, CA) is bound to pre-coated walls of a microcentrifuge tube with a carrier. The procedure of Example 4 was followed. All the CD46 presenting sperm with damaged heads and all somatic cells, mainly white blood cells, and cell debris are bound to the anti-CD46 antibody-carrier complex. The unbound sperm with undamaged intact heads remain in the suspension which is transferred into a new container, ready for further use.

### Example 9: Separation of sperm with undamaged intact heads from sperm with damaged heads from the bull (Taurus sp.) ejaculate

The anti-bull CD46 antibody (clone MEM-258, ABIN94152, Antibodies Online, Aachen, Germany) is bound to magnetic beads as a carrier. The procedure of Example 4 was followed. All the CD46 presenting sperm with damaged heads and all somatic cells, mainly white blood cells, and cell debris are bound to the anti-CD46 antibody-carrier complex and further separated by magnetic field, using a magnetic stand. The unbound sperm with undamaged intact heads remain in the suspension.

### Example 10: Separation of sperm with undamaged intact heads from sperm with damaged heads from the horse (Equus sp.) ejaculate

The anti-horse CD46 antibody (ab231984, Abcam, Cambridge, UK) is bound to polyacrylamide beads. The procedure of Example 4 was followed. All the CD46 presenting sperm with damaged heads and all somatic cells, mainly white blood cells, and cell debris are bound to the anti-CD46 antibody-carrier complex and further separated by centrifugation. The unbound sperm with undamaged intact heads remain in the suspension.

### Example 11: Separation of sperm with undamaged intact heads from sperm with damaged heads from the canine (Canis sp.) ejaculate

The anti-dog CD46 antibody (E4.3, Santa Cruz Biotechnology, Inc., Santa Cruz, CA) is bound to magnetic beads as a carrier. The procedure of Example 4 was followed. All the CD46 presenting sperm with damaged heads and all somatic cells, mainly white blood cells, and cell debris are bound to the anti-CD46 antibody-carrier complex and further separated by magnetic field. The unbound sperm with undamaged intact heads remain in the suspension.

Example 12: Separation of sperm with undamaged and intact heads from sperm with damaged heads in sperm sample from the cryopreserved pig (Sus sp.) semen sample

The procedure of Example 5 was followed, using the antibody and carrier as specified in Example 8. All the CD46 presenting sperm with damaged heads and all somatic cells, mainly white blood cells, and cell debris are bound to the anti-CD46 antibody-carrier complex. The unbound sperm with undamaged intact heads remain in the suspension which is transferred into another container, and they are ready to be used for short or long-term storage under any conditions and media such as cryopreservation or any kind of sample preservation.

### Example 13: Separation of sperm with undamaged and intact heads from sperm with damaged heads from the cryopreserved bull (Taurus sp.) semen sample

The procedure of Example 5 was followed, using the antibody and carrier as specified in Example 9. All the CD46 presenting sperm with damaged heads and all somatic cells, mainly white blood cells, and cell debris are bound to the anti-CD46 antibody-carrier complex and further separated by magnetic field. The unbound sperm with undamaged intact heads remain in the suspension and they are ready to be used for short or long-term storage under any conditions and media such as cryopreservation or any kind of sample preservation.

### Example 14: Separation of sperm with undamaged and intact heads from sperm with damaged heads from the sperm sample of zebra finch (Taeniopygia guttata)

The polyclonal antibody produced according to Example 3 was bound to magnetic beads and was used in the procedure of Example 4. All the CD46 presenting sperm with damaged heads and all somatic cells, mainly white blood cells, and cell debris are bound to the anti-CD46 antibody-carrier complex and separated by magnetic field, using a magnetic stand.

### Example 15: Sperm motility analysis (Figure 9)

Material: Sperm sample after thawing (before selection) or after selection.

Method: Motility was measured with a Computer Assisted Sperm Analysis (CASA) system (ISAS, Proiser, Valencia, Spain). Total motility and progressive motility were evaluated based on manufacturer's setting (thresholds were VAP 10 µm.s⁻and STR 80% for motile and progressively motile sperm, respectively). A 2 µL drop of each semen sample was placed in a 37°C prewarmed Leja counting chamber (IMV Technologies, France). Six fields per sample were evaluated at 100× magnification negative phase-contrast objective. Constant temperature during analysis was ensured by a heating plate prewarmed to 37°C as a part of microscope. The evaluation was based on the analysis of 60 consecutive digitized images, which were taken at a time lapse of 1 s with a camera at a frequency of 60 fps. At least 100 trajectories were analysed per field.

### Example 16: Sperm acrosomal damage detection by CD46 antibody with fluorescent tag using fluorescent microscopy (Figure 5)

Material: Sperm sample after thawing (before selection) or after selection.

Method: Sperm samples were washed twice (300 g, 5 min) in PBS. Pellet was diluted in 100 µL of PBS buffer and 10 µL of mouse monoclonal anti-CD46 antibody (clone M177, ThermoFisher Scientific, USA) conjugated with fluorescence tag (FITC). After 30 minutes incubation in room temperature, spermatozoa were washed in PBS. 9 µL of sperm suspension were transferred onto slide and gently mixed with 3 µL of Vectashield/DAPI (Vector Laboratories Ltd., Peterborough, UK) and mounted under a coverslip. Images were captured in magnification 600x by camera as part of epifluorescent microscope Nikon Eclipse E600 (Tokyo, Japan) and analysed by software NIS Elements (Laboratory Imaging, Inc., Prague, Czech Republic).

### Example 17: Sperm acrosomal damage examined by CD46 antibody using Flow Cytometry (Figure 6)

Material: Sperm sample after thawing or native liquified ejaculate.

Method: Sperm samples were washed twice (300 g, 5 min) in PBS. Sperm concentration was adjusted by PBS to 5 - 10 × 10⁶/mL. 10 µL of mouse monoclonal anti-CD46 antibody (clone M177, ThermoFisher Scientific, USA) conjugated with fluorescence tag (TRITC) was added into 100 µL aliquot. After 30 minutes incubation in room temperature, spermatozoa were washed twice in PBS. Sperm pellet was resuspended in 500 µL of PBS and samples were analysed by BD LSR FortessaTM SORP (BD Biosciences, San Jose, CA, USA). Based on fluorescence signal sperm was divided to CD46+ (Acrosome damaged) and CD46- (Acrosome intact).

### Example 18: Sperm DNA Integrity analysis (Figure 10)

Material: Sperm sample after thawing sorted for damaged (CD46 positive) and intact (CD46 negative) sperm fraction.

Method: For sperm DNA integrity analysis (TUNEL) Apo-direct Kit (Phoenix Flow System, USA) was used. Spermatozoa were washed two times 250 x g for 5 min. Afterwards, 1 mL of Intracellular Perm Wash Buffer (Biolegend, San Diego, USA) was added to the pellet and suspension was incubated for 30 min in 4°C, centrifuged and washed two times. Sperm pellet was fixed in 1 mL of ice-cold 70% ethanol, vortexed and incubated for 30 min in 4°C. After fixation samples were centrifuged 300 x g, 10 min, vortexed and washed two times (300 x g, 10 minutes) in Wash buffer. Thereafter, a reaction mix was added for 60 min incubation in 37°C. Samples were washed twice with Rinse Buffer (300 x g 5 min) and 0.5 mL of Propidium iodide/RNAse A free was added for 30 min and samples were proceeded for analysis. Samples were analysed by flow cytometer BD LSR FortessaTM SORP, BD Biosciences, San Jose, CA, USA. For each sample at least 15000 events were recorded.

### Industrial Applicability

The present invention is a technology for selection and separation of CD46 presenting components from cells not presenting CD46 in cell samples obtained from an animal. The cells not presenting CD46 are sperm with undamaged intact heads. The technology may thus serve for separation of sperm with undamaged intact heads from sperm with damaged heads, as well as from somatic cells and cell debris in a sperm sample. This technology is designed to separate sperm with undamaged intact heads, suitable for use in assisted reproduction, medicine (in particular breeding programs and veterinary medicine) and research. The invention results in an increased quality of the sperm cell sample, as well as allows separation of somatic cells and/or debris from sperm-containing samples. Such purified samples result in an increased fertilization or medical or research success. The invention also results in an increased quality of the sperm sample for long- or short-term storage under any condition and media such as cryopreservation. This invention also saves large amount of cryoprotectives and plastic by storage of the pre-selected quality sample.

## Claims

1. A method of separation of sperm cells with undamaged intact heads from sperm cells with damaged heads and/or somatic cells and/or cell debris in a sperm sample obtained from an animal, comprising the steps of:
- providing an anti-CD46 antibody bound to a carrier and/or to a tag;
- contacting the sperm sample with the anti-CD46 antibody bound to the carrier and/or to the tag in order to bind CD46 presenting cells and/or cell debris from the sperm sample to the anti-CD46 antibody ;
- removing the CD46 presenting cells bound via the anti-CD46 antibody to the carrier and/or to the tag to obtain a sperm sample free of CD46 presenting cells, wherein CD46 presenting cells include sperm cells with damaged heads, somatic cells and cell debris.

2. The method according to claim 1, wherein the sperm sample free of CD46 presenting cells is a sperm containing substantially only sperm cells with undamaged intact heads.

3. The method according to claim 1 or 2, wherein the starting sperm sample is an ejaculate or a pre-treated ejaculate or a semen sample or a pre-treated semen sample, wherein the pre-treatment preferably includes removal of seminal fluid and/or tissues and/or cell debris from the ejaculate.

4. The method according to any one of the preceding claims, which contains the following steps:
- providing at least one immunization peptide for anti-CD46 antibodies production ;
- producing an anti-CD46 antibody;
- binding the anti-CD46 antibody to a carrier and/or to a tag;
- contacting the sperm sample with the anti-CD46 antibody bound to the carrier and/or to the tag with a sperm sample in order to bind CD46 presenting cells, including sperm cells with damaged heads and/or somatic cells and/or cell debris, from the sperm sample to the anti-CD46 antibody;
- separating the sperm cells with damaged heads and/or somatic cells and/or cell debris, bound via the anti-CD46 antibody to the carrier and/or to the tag, from the sperm sample;
thereby obtaining two cell fractions: 1) sperm cells with undamaged intact heads and 2) sperm cells with damaged heads and/or somatic cells and/or cell debris.

5. The method according to claim 4, wherein the anti-CD46 antibody is produced by immunization of a production animal by an immunization peptide having the sequence PFEAMELKGTPKLY (SEQ ID NO. 1).

6. The method according to any one of the preceding claims, wherein the carrier is selected from the group comprising beads, magnetic beads, polymeric substrates, cellulosic substrates, metallic substrates, magnetic or magnetizable carriers.

7. The method according to any one of claims 1-5, wherein the carrier is a vessel, such as a well, well plate, beaker, capillary or test tube, wherein the anti-CD46 antibody is coated or immobilized on the inner surface of the vessel; or wherein the carrier is coated or immobilized on the inner surface of a vessel, such as a well, well plate, beaker, capillary or test tube.

8. The method according to any one of the preceding claims, wherein the removal of the CD46 presenting cells bound via anti-CD46 antibody to the carrier and/or to the tag is carried out by centrifugation, sedimentation, flow separation methods such as FACS, capillary flow separation, separation on chips, or by use of magnetic force such as MACS or magnetic separation.

9. Use of anti-CD46 antibody for *in vitro* separation of sperm cells with undamaged intact heads from sperm cells with damaged heads and/or somatic cells and/or cell debris in a sperm sample obtained from an animal.

## Patentansprüche

1. Verfahren zur Trennung von Spermien mit unbeschädigten intakten Köpfen von Spermien mit beschädigten Köpfen und/oder somatischen Zellen und/oder Zelltrümmern in einer von einem Tier gewonnenen Spermienprobe, umfassend die Schritte:
- Bereitstellen eines an einen Träger und/oder eine Markierung gebundenen Anti-CD46-Antikörpers;
- Inkontaktbringen der Spermienprobe mit dem an den Träger und/oder die Markierung gebundenen Anti-CD46-Antikörper, um CD46-präsentierende Zellen und/oder Zelltrümmer aus der Spermienprobe an den Anti-CD46-Antikörper zu binden;
- Entfernen der über den Anti-CD46-Antikörper an den Träger und/oder die Markierung gebundenen CD46-präsentierenden Zellen, um eine Spermienprobe zu erhalten, die frei von CD46-präsentierenden Zellen ist, wobei CD46-präsentierende Zellen Spermien mit beschädigten Köpfen, somatische Zellen und Zelltrümmer umfassen.

2. Verfahren nach Anspruch 1, wobei die Spermienprobe, die frei von CD46-präsentierenden Zellen ist, ein Spermium ist, das im Wesentlichen nur Spermien mit unbeschädigten intakten Köpfen enthält.

3. Verfahren nach Anspruch 1 oder 2, wobei die Ausgangsspermaprobe ein Ejakulat oder ein vorbehandeltes Ejakulat oder eine Samenprobe oder eine vorbehandelte Samenprobe ist, wobei die Vorbehandlung vorzugsweise die Entfernung von Samenflüssigkeit und/oder Gewebe und/oder Zelltrümmern aus dem Ejakulat umfasst.

4. Verfahren nach einem der vorhergehenden Ansprüche, das die folgenden Schritte umfasst:
- Bereitstellen von mindestens einem Immunisierungspeptid zur Produktion von Anti-CD46-Antikörpern;
- Herstellen eines Anti-CD46-Antikörpers;
- Binden des Anti-CD46-Antikörpers an einen Träger und/oder an eine Markierung;
- Inkontaktbringen der Spermaprobe mit dem an den Träger und/oder an die Markierung gebundenen Anti-CD46-Antikörper mit einer Spermaprobe, um CD46-präsentierende Zellen, einschließlich Spermien mit beschädigten Köpfen und/oder somatische Zellen und/oder Zelltrümmer, aus der Spermaprobe an den Anti-CD46-Antikörper zu binden;
- Abtrennen der Spermien mit beschädigten Köpfen und/oder somatischen Zellen und/oder Zelltrümmern, die über den Anti-CD46-Antikörper an den Träger und/oder an die Markierung gebunden sind, von der Spermienprobe;
wodurch zwei Zellfraktionen erhalten werden: 1) Spermien mit unbeschädigten intakten Köpfen und 2) Spermien mit beschädigten Köpfen und/oder somatischen Zellen und/oder Zelltrümmern.

5. Verfahren nach Anspruch 4, wobei der Anti-CD46-Antikörper durch Immunisierung eines Nutztiers mit einem Immunisierungspeptid mit der Sequenz PFEAMELKGTPKLY (SEQ ID NO. 1) erzeugt wird.

6. Verfahren nach einem der vorhergehenden Ansprüche, wobei der Träger aus der Gruppe ausgewählt ist, die Perlen, magnetische Perlen, Polymersubstrate, Zellulosesubstrate, metallische Substrate, magnetische oder magnetisierbare Träger umfasst.

7. Verfahren nach einem der Ansprüche 1 bis 5, wobei der Träger ein Gefäß ist, wie beispielsweise eine Vertiefung, eine Vertiefungsplatte, ein Becher, eine Kapillare oder ein Reagenzglas, wobei der Anti-CD46-Antikörper auf der Innenfläche des Gefäßes beschichtet oder immobilisiert ist; oder wobei der Träger auf der Innenfläche eines Gefäßes beschichtet oder immobilisiert ist, wie beispielsweise eine Vertiefung, eine Vertiefungsplatte, ein Becher, eine Kapillare oder ein Reagenzglas.

8. Verfahren nach einem der vorhergehenden Ansprüche, wobei die Entfernung der CD46-präsentierenden Zellen, die über den Anti-CD46-Antikörper an den Träger und/oder an die Markierung gebunden sind, durch Zentrifugation, Sedimentation, Durchflusstrennungsverfahren wie FACS, Kapillarflusstrennung, Trennung auf Chips oder durch Verwendung magnetischer Kraft wie MACS oder magnetische Trennung durchgeführt wird.

9. Verwendung des Anti-CD46-Antikörpers zur *In-vitro-Trennung* von Spermienzellen mit unbeschädigten intakten Köpfen von Spermienzellen mit beschädigten Köpfen und/oder somatischen Zellen und/oder Zelltrümmern in einer von einem Tier gewonnenen Spermienprobe.

## Revendications

1. Procédé de séparation de spermatozoïdes à tête intacte non endommagée des spermatozoïdes à tête endommagée et/ou cellules somatiques et/ou débris cellulaires dans un échantillon de sperme obtenu à partir d'un animal, comprenant les étapes consistant à:
- fournir un anticorps anti-CD46 lié à un support et/ou à un marqueur;
- mettre en contact l'échantillon de sperme avec l'anticorps anti-CD46 lié au support et/ou au marqueur afin de lier les cellules présentatrices de CD46 et/ou les débris cellulaires de l'échantillon de sperme à l'anticorps anti-CD46;
- retirer les cellules présentatrices de CD46 liées par l'anticorps anti-CD46 au support et/ou au marqueur pour obtenir un échantillon de sperme sans cellules présentatrices de CD46, les cellules présentatrices de CD46 comprenant des spermatozoïdes à tête endommagée, des cellules somatiques et des débris cellulaires.

2. Procédé selon la revendication 1, où l'échantillon de sperme sans cellules présentatrices de CD46 est un sperme contenant sensiblement uniquement des spermatozoïdes à tête intacte non endommagée.

3. Procédé selon la revendication 1 ou 2, où l'échantillon initial de sperme est un éjaculat ou un éjaculat prétraité ou un échantillon de sperme ou un échantillon de sperme prétraité, le prétraitement comprenant de préférence l'élimination du liquide séminal et/ou des tissus et/ou des débris cellulaires de l'éjaculat.

4. Procédé selon l'une quelconque des revendications précédentes, qui comprend les étapes suivantes :
- fourniture d'au moins un peptide d'immunisation pour la production d'anticorps anti-CD46;
- production d'un anticorps anti-CD46;
- liaison de l'anticorps anti-CD46 à un support et/ou à un marqueur;
- mise en contact de l'échantillon de sperme avec l'anticorps anti-CD46 lié au support et/ou au marqueur avec un échantillon de sperme afin de lier des cellules présentant CD46, y compris des spermatozoïdes avec des têtes endommagées et/ou des cellules somatiques et/ou des débris cellulaires, de l'échantillon de sperme à l'anticorps anti-CD46;
- séparer les spermatozoïdes à têtes endommagées et/ou les cellules somatiques et/ou les débris cellulaires, liés par l'anticorps anti-CD46 au support et/ou au marqueur, de l'échantillon de sperme;
obtenant ainsi deux fractions cellulaires : 1) des spermatozoïdes à têtes intactes non endommagées et 2) des spermatozoïdes à têtes endommagées et/ou des cellules somatiques et/ou des débris cellulaires.

5. Procédé selon la revendication 4, où l'anticorps anti-CD46 est produit par immunisation d'un animal de production par un peptide d'immunisation ayant la séquence PFEAMELKGTPKLY (SEQ ID NO. 1).

6. Procédé selon l'une quelconque des revendications précédentes, où le support est choisi dans le groupe comprenant des billes, des billes magnétiques, des substrats polymères, des substrats cellulosiques, des substrats métalliques, des supports magnétiques ou magnétisables.

7. Procédé selon l'une quelconque des revendications 1 à 5, où le support est un récipient, tel qu'un puits, une plaque à puits, un bécher, un capillaire ou un tube à essai, où l'anticorps anti-CD46 est enduit ou immobilisé sur la surface intérieure du récipient; ou où le support est enduit ou immobilisé sur la surface intérieure d'un récipient, tel qu'un puits, une plaque à puits, un bécher, un capillaire ou un tube à essai.

8. Procédé selon l'une quelconque des revendications précédentes, où l'élimination des cellules présentant CD46 liées par l'anticorps anti-CD46 au support et/ou au marqueur est réalisée par centrifugation, sédimentation, méthodes de séparation par flux telles que FACS, séparation par flux capillaire, séparation sur puces, ou par utilisation de force magnétique telle que MACS ou séparation magnétique.

9. Utilisation d'anticorps anti-CD46 pour la séparation *in vitro* de spermatozoïdes avec des têtes intactes non endommagées de spermatozoïdes avec des têtes endommagées et/ou de cellules somatiques et/ou de débris cellulaires dans un échantillon de sperme obtenu à partir d'un animal.
